(19) Europäisches Patentamt
European Patent Office
Office européen des brevets

(11) **EP 1 443 817 B1**

(12) **EUROPEAN PATENT SPECIFICATION**

(45) Date of publication and mention
of the grant of the patent:
**05.04.2006 Bulletin 2006/14**

(51) Int Cl.:
*A01M 1/20* (2006.01)     *A61L 9/03* (2006.01)
*A61L 9/12* (2006.01)

(21) Application number: **02801392.8**

(22) Date of filing: **11.10.2002**

(86) International application number:
**PCT/GB2002/004638**

(87) International publication number:
**WO 2003/032723 (24.04.2003 Gazette 2003/17)**

(54) **SUBSTANCE DELIVERY DEVICE**

STOFFABGABEVORRICHTUNG

DISPOSITIF DE DISTRIBUTION DE SUBSTANCE

(84) Designated Contracting States:
**AT BE BG CH CY CZ DE DK EE ES FI FR GB GR
IE IT LI LU MC NL PT SE SK TR**

(30) Priority: **16.10.2001 GB 0124730**

(43) Date of publication of application:
**11.08.2004 Bulletin 2004/33**

(73) Proprietor: **Reckitt Benckiser (Australia) Pty
Limited
West Ryde, NSW 2114 (AU)**

(72) Inventor: **MORGAN, John, Douglas, Peter,
Reckitt Benckiser
West Ryde, NSW 2114 (AU)**

(74) Representative: **Bowers, Craig Malcolm
Reckitt Benckiser plc
Intellectual Property Department
Patents Group
Dansom Lane
Hull HU8 7DS (GB)**

(56) References cited:
WO-A-97/12517          FR-A- 1 041 075
FR-A- 2 054 435        US-A- 3 698 974
US-A- 4 158 440        US-A- 4 647 433

## Description

### Field of the invention

The present invention relates to devices for delivering active substances into the atmosphere, such as insecticidal substances or fragrances.

### Background to the invention

Electrically heated insecticide emanator devices provide relief from mosquitos and other insects in many homes around the globe, and when used are preferred to mosquito coils because of the lack of smell, smoke and ash. Electrical devices ("electricals") comprise an electrically powered heater unit and a disposable refill unit which contains the insecticide. Two types of refill unit are commonly available - bottled liquid refills ("liquids") and insecticide impregnated cellulose fibre mats ("mats"). The mats have been available for about thirty years, and offer a single night's protection. The liquids are a more recent innovation, and provide from 30 to 180 nights of protection, depending on the product.

The main benefit that the liquid refills offer over the standard mat is long lasting protection. This comes at a cost - the heater units that accept the liquid refills are different to the mat heaters. Therefore existing mat users must purchase a new device to gain this benefit, and for many of the markets for these products the cost is too high to bear.

These devices are, generically speaking, chemical active substance delivery systems. Their effectiveness depends on knowing the rate of active delivery that is required to achieve the desired level of protection, and engineering the device to deliver at that level. However, it appears that both the understanding of the delivery rates of these devices and the required delivery rates are fairly poor.

The results presented herein show that current insecticidal mats are very inefficient, wasteful delivery systems. The delivery rate of active decays exponentially with time, with a short half life. Therefore, to achieve one whole night's protection, a large amount of active substance must be used. Most of this is released very early in the operation of the mat, but this waste is necessary to achieve activity out to the required time of 12 hours.

Based on the estimated least effective release rate, an efficient delivery system could use 1% of the amount of active presently used, and still provide an effective product.

For the same reason, current mat designs cannot deliver a long lasting mat. The amount of active required increases exponentially with the desired lifetime. The amount of active that would be required for a 7 day mat would make such a product prohibitively expensive.

An efficient delivery system is therefore needed.

US 4,158,440 describes a device for controlled release of a volatile substance. The device comprises an absorbent material and a layer of ultramicroporous material such as gelled cellulose triocetate in an impermeable envelope having a hole through which the ultramicroporous material is exposed.

### Summary of the Invention

We have characterised the kinetics and mechanisms involved in the release of volatile active substances from a number of substrates of varying configurations, including commercially available insecticidal mats.

We have shown that sealing the substrate that contains the active substances with an outer layer that is impermeable to the active substance and providing only a limited area of release by means of apertures in the surface of the outer layer of relatively small total surface area as compared with the surface area of the substrate, results in substantially linear release characteristics as compared with the exponential release characteristics seen with existing substance delivery devices.

The devices of the present invention are of simple construction and do not require additional semi-permeable membrane layers or mechanisms of complex physical construction to achieve the desired delivery characteristics.

Accordingly, the present invention according to claim 1 provides a substance delivery device comprising (i) a substrate layer impregnated with one or more active substances, the substrate layer being in direct fluid communication with the atmosphere, (ii) an outer layer which encloses the substrate layer and is substantially impermeable to the one or more active substances, and (iii) one or more apertures in the outer layer which permit release of the one or more active substances from the substrate layer into the atmosphere, the apertures being present only in a portion of the outer layer which in use is exposed to the atmosphere, wherein the total surface area of the apertures is less than 2% of the total surface area of the outer layer such that the one or more active substances are released into the atmosphere at a substantially linear rate.

The apertures constitute less than 2% or, preferably, 1% of the surf ace area of the outer layer.

Although in some embodiments, there may be a plurality of apertures, it is preferred that there is only one aperture in the material. The apertures may advantageously be substantially in the centre of said portion.

In one embodiment, the substrate comprises one or more holes which are in at least partial alignment with the one or more apertures. The holes may extend partially through the substrate layer. Alternatively, the holes may extend completely through the substrate layer.

In a preferred embodiment, the substrate layer is substantially flat, such as a fibrous mat.

The one or more active substances may be, for example an insecticidal substance (such as a pyrethroid), a fragrance or other volatile.

Preferably, the solid substrate comprises one or more solvents. More preferably, at least one of the solvents has a molar enthalpy of vaporisation of greater than 9 kcal/mol. The substrate layer may also comprise an indicator layer comprising a material the light transmitting properties of which vary depending on the amount of solvent present in the substrate.

The solid substrate may also comprise one or more surfactants, dyes, fragrances, preservatives and/or antioxidants etc,

In preferred embodiments, the device further comprises removable sealing means for sealing said apertures prior to use.

The present invention further provides a method according to claim 17 for producing a device of the invention which method comprises enclosing/sealing a substrate layer comprising one or more active substances in an outer layer which is impermeable to the one or more substances; and introducing one or more apertures in the material.

The present inventon further provides a method according to claim 18 for producing a device of the invention which methods comprises enclosing/sealing a solid substrate comprising one or more active substances in an outer layer which is impermeable to the one or more substances and which comprises one or more apertures.

The above methods may further comprise sealing said one or more apertures with a removable sealing means.

In particular, the devices of the present invention may be used with heating means, such as an electrical heating device.

Thus, the present invention also provides a method according to claim 20 for releasing one or more active substances into the atmosphere which method comprises heating the substrate layer of the device of the invention so as to cause release of the one or more active substances into the atmosphere via the apertures in the outer layer.

The present invention also provides a method according to claim 21 for inhibiting insect biting which method comprises heating the substrate layer of the device of the invention so as to cause release of one or more insecticidal substances in the substrate into the atmosphere via the apertures in the material of the device.

Detailed description of the invention

Substrate layer and enclosing materials

The substrate which comprises one or more active substances may be of any shape. However, it is particularly convenient for the substrate to be substantially flat, for example, in the form of a mat used in conventional electrically heated insecticide emanator devices. The substrate may be of any size. However, a substrate size that is compatible with existing electrically heated insecticide emanator devices may conveniently be used.

The substrate is generally a solid substrate made of a material which can be impregnated with an active substance such as an insecticidal substance. Suitable materials are typically those that are absorbent and allow migration/flow of the active substances throughout the material. Examples of suitable materials include fibrous materials such as cellulose fibre, cardboard, paperboard, sponge, paper, fibreboard, cloth, sintered glass, glass fibre, metal fibre, asbestos, sintered metal and sintered plastic.

The substrate may be a single layer or a plurality of layers. The layers made be made of the same or different materials. In addition, the substrate may comprise one or more layers made of a material which is substantially impermeable to the one or more active substances. These impermeable layers are internal to the substrate i.e. the outermost layers of the substrate will be permeable to the active substances and solvents. Such impermeable materials are described below in relation to the material which encloses the solid substrate.

In one embodiment, the substrate may also comprise an indicator layer comprising a material, the light transmitting properties of which vary depending on the amount of solvent present in the substrate e.g. such that as the solvent is exhausted the indicator layer becomes more opaque. Examples of such materials include phase inversion membranes such as polyvinylidene fluoride, polytetrafluoroethylene, nylon, polysulfones such as polyphenylethersulfone (i.e. poly(oxy-1,4-phenylenesulfonyl-1,4-phenylene).

The solid substrate is enclosed by an outer layer made of a material which is substantially impermeable to the one or more active substances. Preferably the material, in the absence of any apertures, is completely impermeable to the one or more active substances. Suitable materials include flexible materials such as metal foils and polymer films such as metalized plastic sheets that can be heat sealed, as well as rigid materials such as metals, plastics or glass. The material should be compatible with the one or more active substances and solvents etc. contained in the solid substrate. Where, in use, the one or more active substances are volatised by heat generated by a heating means, the material will need to be resistant to such heat.

The material should completely enclose the substrate, with the exception of the apertures present in the material, to allow release of the one or more active substances into the atmosphere when desired. Thus, the term "enclosed" as used herein, means that in the absence of any apertures, the substrate layer is completely sealed by the outer layer. Furthermore, the outer layer is positioned in relation to the substrate layer such that the outer layer direct contacts the substrate layer over substantially the entire surface area of the substrate layer. Thus, there are no other layers between the outer layer and the substrate, such as layers of semi-permeable materials.

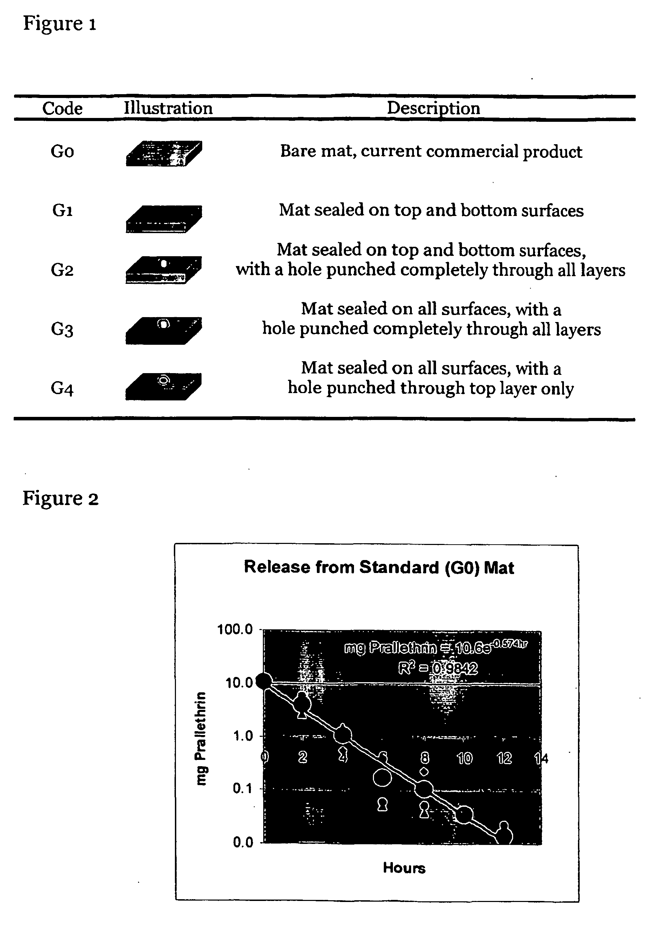

In the assembled device of the invention, the outer layer will comprise one or more apertures in fluid communication with the substrate layer. Since there are no other layers or barriers between the surface of the substrate and the apertures, the substrate can be considered to be in direct fluid communication with the apertures and the atmosphere. This contrasts with previously known devices where a layer of a permeable or semi-permeable material is placed between the substrate layers. The apertures are present only on a surface of the device which when in use is exposed to the atmosphere, i.e. typically uppermost to the atmosphere (see for example the apertures shown in Figure 1). Typically, where more than one aperture is present, all of the apertures are on the same surface, that surface being the surface which when in use is exposed to the atmosphere. Our results show that apertures present on other surfaces, such as around the sides, result in higher rates of release of the one or more active substances, which may lead to sub-optimal release kinetics in relation to extended useful life for the device of the invention. Furthermore, in the case of planar substrates with opposed surfaces, we have shown that apertures on both surfaces also lead to sub-optimal release kinetics. Consequently, where the substrate is planar, the apertures should be present on only one surface, that surface being the surface that when in use is exposed to the atmosphere.

Accordingly, in a highly preferred embodiment, the one or more apertures are present only on the exposed surface.

The total surface area of the apertures is of a size such that substantially linear release of the one or more active substances from the substrate layer is achieved when the device is in use. The total surface area of the apertures is therefore small in relation to the total surface area of the substrate, typically less than 2, 1 or 0.5% of the total surface area of the substrate. Most preferably, the total surface area is less than 1% of the total surface area of the substrate. Since the apertures are made in the outer layer, the surface area may also be calculated in relation to the outer layer, which will be very similar to that of the substrate due to the design of the devices. Thus the total surface area of the apertures is typically less than 2,1 or 0.5% of the total surface area of the outer layer. Most preferably, the total surface area is less than 1% or 0.5% of the total surface area of the outer layer.

Another way of expressing the percentage surface area of the apertures is in relation to the surface area of the outer layer which when in use is exposed to the atmosphere, However, for simplicity it is preferred to calculate the percentage based on the total surface area of the outer layer/substrate layer.

The relatively small surface area of the apertures is required to achieve the linear delivery characteristics of the devices of the present invention since exposure of a large surface area of the substrate to the atmosphere allows direct release of active substance over a large area, resulting in exponential release kinetics. By contrast, restricting the exposed regions to small apertures with a small total surface area results in a controlled release of the active substances since the majority of the active substance needs to move from the unexposed regions to the aperture(s) before delivery by evaporation can occur.

Although a number of apertures may be present in the material, it is preferred that there are fewer than 10 apertures, such as only one or two apertures. Further, in a preferred embodiment, where there is only one aperture, the aperture is located substantially centrally in the exposed surface (as is shown for example in Figure 1) . In another embodiment there may be two or more apertures located off-centre, for example near the edge of a substantially flat solid substrate, typically in a symmetrical configuration.

In addition to the apertures in the outer layer which encloses the substrate, it may be desirable for the substrate to comprise one or more holes. The holes will typically be at least partially aligned, preferably substantially aligned, with a corresponding aperture in the material once the device has been assembled. The holes may extend partially, or completely, through the substrate and may be of any suitable geometry However, it is preferred that at least one hole extends only partially and not completely through the substrate particularly where the hole is aligned with an aperture which is centrally located. Where the substrate has a plurality of layers, at least one of which is impermeable to the one or more active substances, the hole may extend through the substrate such that there is a hole completely through the impermeable layer to allow the allow layers to be in fluid contact with one another.

Overall, it is preferred that the configuration of apertures is such that substantially constant release (zero order kinetics) of the one or more active substances is achieved.

In one embodiment, the substrate comprises one or more holes, or discontinuities in the substrate, which are not aligned with a corresponding aperture, i.e. are not exposed to the atmosphere. These holes may be used to modulate the flow of solvent/active substance towards regions of the substrate that are aligned with corresponding apertures in the impermeable outer layer material. For example, in the case of a flat mat, the two ends of the mat may have a portion cut out of the substrate such that solvent/active substance will flow around the resulting gap.

The device of the invention, comprising the substrate enclosed by the impermeable material may also comprise a sealing means to ensure that the active substances are not released into the atmosphere prior to use. Consumer products of this type are required to have a long shelf-life and it will therefore typically be necessary to supply the product completely sealed to prevent loss of active substances and volatile solvents in storage. The sealing means will typically be an adhesive polymer film covering the apertures that can be peeled off by the user prior to first use.

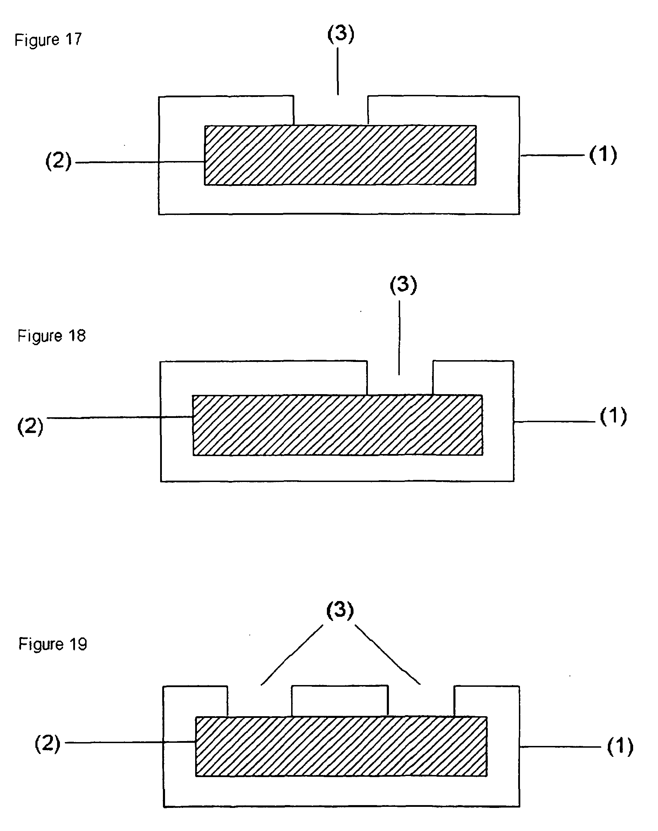

Particular embodiments of the devices of the present invention are shown in Figures 17, 18 and 19. These devices are substantially flat with a single substrate layer (2) completely enclosed by a single outer layer (1) which has a single or double aperture (3).

Active substances and solvent systems

The one or more active substances may be selected from any substance which it is desired to deliver to the atmosphere using the devices of the invention. Suitable active substances include fragrances, acaricides, repellents, spatial bactericides and antibacterials such as hinokitiol, linalool, citral, pinene, menthol, terpene alcohols, etc., fungicides and insecticidal substances such as insecticidal pyrethroids. Examples of pyrethroids include prallethrin, allethrin, s-bioallethrin, furamethrin, tefuramethrin (5-propargyl-2-furyl methyl 2,2,3,3,-tetramethylcyclopropanecarboxylate), terallethrin, empenthrin, 5-propargyl-2-methyl-3-furylmethyl 2,2,3,3-tetramethyl cyclopropane carboxylate and fenfluthrin. The above may be used individually or in combination.

The amount of active substance present in the substrate is typically in the range of from 0.5 mg to 50 mg. For example, a standard size mat may comprise from 5 to 50 mg of an active substance. However, the actual amount of active substance needed to achieve the desired effect over the desired period will vary from substance to substance.

For application to the substrate, the active substances are typically dissolved in a compatible solvent, such as a mixture of isopropyl myristate and deodorised kerosene, or mixed with a solvent carrier to form a suspension or emulsion. Preferably, at least one of the solvent components is of low volatility such that there is still solvent in the substrate over the lifetime of the device in use. more specifically, it is preferred that at least one of the solvents has a molar enthalpy of vaporisation of greater that $3.77 \times 10^3$ J/mol (9 kcal/mol) preferably greater than $5.02 \times 10^3$ J/mol (12-kcal/mol), most preferably greater than $6.07 \times 10^3$ J/mol (14.5 kcal/mol).

In one embodiment, the volatility of at least one of the solvents is similar to that of the one or more active substances.

Kerosene is highly volatile and tends to be lost rapidly from the substrate. However, we have show that isopropyl myristate has a much slower rate of evaporation and in preferred embodiments of the present invention evaporates at essentially a linear rate. We have also shown that where the isopropyl myristate has a slightly faster rate of evaporation than the active substance, the concentration of the active substance at the apertures increases over time.

Thus the delivery of the active substances can be regulated by the characteristics and/or amount of the solvent. For example, possibilities include varying the viscosity of the solvent, changing the type of solvent (i.e. using solvents with different volatilities) and/or including a surfactant such as polyglycerol oleate to flatten surface tension gradients.

Accordingly, the teachings presented herein allow the skilled person to design a device with the desired lifetime and rate of delivery of active substance by preparing a solid substrate impregnated with varying amounts and types of solvent and active substances and enclosing/sealing the solid substrate in a material impermeable to the active substances which material comprises at least one aperture.

A particular benefit of the devices of the present invention is that they be designed to provide cost-effective delivery of active substances over periods of between 2 and 30 days, periods which are not currently catered for by existing mat-type devices, because too much active substance would be needed, or liquid emanator devices because they are not economical over that time period.

Manufacture of devices

The devices of the invention may typically be manufactured in one of two main ways. In one embodiment, the solid substrate may be sealed inside the material and then the material pierced or punched to produce one or more apertures. At the same time, if holes in the solid substrate are required, the means used to produce the one or more apertures may be used to produce holes in the substrate. In this way, the holes will be substantially aligned with the apertures.

In another embodiment, apertures are made in the material, or the material is formed so as to already include apertures, and the solid substrate is then sealed within the material. For example, in the case of a mat substrate, the two different layers of material may be used to seal the substrate, one for the top surface of the substrate and one for the bottom layer. In a preferred embodiment, the top layer only comprises apertures. The substrate is placed between the top and bottom layers of material and the two layers sealed together, such as by heat-sealing means or adhesive.

Generally, the active substances, typically dissolved in one or more solvents, are applied to the solid substrate and then the impregnated substrate sealed within the material as described. Surfactants and other optional ingredients may also be applied to the substrate prior to sealing.

Once the substrate has been sealed within the material, removable sealing means, such as an adhesive polymer film, are typically applied to the apertures to completely seal the substrate.

Use of the devices

The devices of the invention are designed for the release of active substances of a period of time typically greater than seven days. Uses include freshening air in a confined space and as an insecticidal device, particularly to inhibit insect biting activity. The devices of the invention may be used in electrical heat-transpiration devices/diffusers where the device of the invention is inserted into a housing which comprises heating means. When the electrical device is connected to the electrical supply, the heating means heat the solid substrate causing release of the active substances into the atmosphere via the apertures. The heating means typically provides a heating temperature of from 70 to 170°C .

Where a removable sealing means is present on a device of the invention, the removable sealing means is removed prior to insertion into the housing of the electrical device.

The devices of the invention may be used in existing electrical emanator devices which are designed for use with mats, or otherwise.

However, the devices of the invention need not be used with heating means where the volatility of the active substances/solvents is such that heating is not required to obtain effective release of the active substance into the atmosphere, i.e. the devices function at ambient temperature.

The present invention will now be described further with reference to the following Examples, which are illustrative only and non-limiting. The Examples refer to Figures:

Description of the Figures

**Figure 1** - Insect mat designs

**Figure 2.** Amount of active remaining on the G0 mat after heating for a given time. The decay is a single exponential with time constant 0.547 hr$^{-1}$. Large circles are the averaged data, small symbols are the individual mat results.

**Figure 3.** Amount of active remaining on G1, G2 and G3 mats after heating for a given time. The time units are eight hour 'nights'. In the regression equations x = 8 hours. Each data point is a single replicate.

**Figure 4.** Amount of active remaining on G4 mats after heating for a given time. The time units are eight hour 'nights'. In the regression equations $x$ = 8 hours. Each data point is a single replicate. Note that the scale is linear, not logarithmic.

**Figure 5.** Amount of active remaining on G5 mats (hole at edge) after heating for a given time. The time units are twelve hour 'nights'.

**Figure 6.** Amount of active remaining on G6 mats (two holes) after heating for a given time. The time units are twelve hour 'nights'.

**Figure 7**. Contour plot of active concentration through the $x$-$y$ plane of an unheated mat. The active is initially distributed uniformly.

**Figure 8.** Contour plot of active concentration through the $x$-$y$ plane of an unsealed (G0) mat after 2 hours heating. A small gradient in one direction is evident, but no significant preferential loss from corners or edges.

**Figure 9.** Contour plot of active concentration through the $x$-$y$ plane of a sealed (G4) mat after 24 hours heating. The distribution is highly peaked beneath the hole.

**Figure 10.** Amount of active and two solvents remaining on G4 mat over 7x8hr nights.

**Figure 11.** Mass ratio of prallethrin to IPM through the life of the mat.

**Figure 12.** Effect of active loading on release rate for the G4 mat.

**Figure 13.** KD50 against *Culex pipiens vs.* heating time for commercial 10 mg prallethrin mats.

**Figure 14.** KD50 *vs.* 8 hours 'nights' for the 10 mg G4 design, for *Aedes aegypti.* Efficacy is constant up to 6 nights and drops on the seventh, as might be expected from the release profile of Figure 4.

**Figure 15.** Bioefficacy of the G4 mat *vs Culex quinquefasciatus.* Graph shows KD50 over 7x8hr nights.

**Figure 16.** Bioefficacy of the G4 mat *vs Culex quinquefasciatus.* Graph shows 24hr mortality over 7x8hr nights.

**Figures 17, 18 , 19** show diagrammatic representations of particular embodiments of the invention (corresponding to the G4, G5 and G6 mats).

EXAMPLES

**Materials and Methods**

Most of the materials used to prepare the experimental prototypes in this study were sourced from the Reckitt Benckiser factories and laboratories, and are typically the materials used in preparation of the commercial articles.
Insecticidal mats were prepared by treating small compressed cellulose fibre mats with an insecticide solution. These mats measured 35 x 22 x 2 mm, weighed approximately 0.85 g and were branded "Shieldtox". Each mat was dosed using a Transferpette with $120\pm2$ mg of the insecticide solution of Table 1, to give a mat containing 10.0 mg prallethrin. The solution took several hours to wet throughout the mat, but ultimately became uniformly distributed. The resulting article is identical to the commercial "King Mat" product.

**Table 1.** Insecticide formulation for mats.

| Ingredient | Percenta ge | Description |
|---|---|---|
| Prallethrin 90% | 9.26 | Technical grade ex Sumitomo, |
| Exxsol D80 | 23.94 | Dearomatized kerosene |
| Isopropyl Myristate | 61.20 | High boiling point solvent |
| t-Butylhydroxytoluene | 5.00 | Antioxidant |
| oilsol Red | 0.60 | Oil soluble red dye |

The treated mats were sealed in a number of ways, presenting mass transport barriers of different geometries. The bare, unsealed mat is referred to as "geometry zero", or G0. More geometries were prepared by sealing the treated mats in adhesive backed metal tape, or with heat sealable packaging foil.
The first geometry, G1, consisted of a mat with metal tape applied to the top and bottom surfaces, forming a sandwich, with the edges open. G2 was the same as G1 save for a 3 mm diameter hole punched through the middle of the entire assembly. G3 consisted of a mat completely sealed within metal tape - top, bottom and all edges, but with a 3 mm hole punched directly through the whole assembly. Holes were punched using a handheld leather punching tool.
G4 was prepared using the plastic laminate that is normally used for packaging the consumer article. This is a metallised plastic sheet that can be heat sealed, by pressing two sheets together in a heated crimper. G4 was prepared by sandwiching a mat between two sheets of laminate and heat sealing by crimping very close to the edges of the mat, thus completely sealing the mat. A 3 mm diameter hole was cut in the middle of the top sheet only (not completely through as in G2 and G3) to expose the mat underneath.
These various designs are summarised in Figure 1.
Two variants of the G4 design were also examined. The G5 design was identical to the G4 design save for the placement of the hole in the top sheet. Instead of being in the middle of the mat it was cut such that the centre of the hole was 2.5 mm from one of the shorter edges and equidistant from each of the longer edges. The G6 design was identical to the G4 design save for the placement and number of holes in the top sheet. Instead of having one hole in the middle of the mat, two holes were cut such that the centre of each hole was 2.5 mm from one of the shorter edges and equidistant from each of the longer edges.
The G0, G1, G2 and G3 mats are not within the scope of the present invention whereas the G4, G5 and G6 represent particular embodiments of the devices of the present invention.

The active content of fresh and used mats was determined by chopping up the mat and any associated packaging, sonicating in acetone and analysing for active by gas chromatography (Hewlett Packard 6890, Reckitt Benckiser Test Method 31385).

Bioefficacy was determined in some experiments as the KD50 (time to knock down 50% of a population) versus a given mosquito species in a test chamber. Mosquito species were Aedes *aegypti* and *Culex quinquefasciatus.* The test chamber dimensions were 70x70x70 cm (0.343 m$^3$). The mats were heated in the heater units in a fume hood for 1 hour prior to testing, then introduced to the test chamber for 3.0 minutes, then removed. Approximately 20 mosquitoes were then introduced to the chamber, and the number of knockdowns were recorded at suitable intervals. KD50s were determined by probit analysis based on 5 replicates per treatment.

Bioefficacy was also determined by measuring the inhibition of landing and biting by *Aedes Aegypti.* Biting and landing inhibition was measured in a ventilated chamber of 29 cubic metres, divided in half by a wall containing a stable door, the top half of which is open during the test. A human volunteer sits on one side of the wall with the test device, and several hundred mosquitoes are released on the other side. The bite inhibition is determined as the average percentage reduction in the number of bites relative to a control with no device operating, based on five replicates. The landing inhibition is determined as the average percentage reduction in the number of landings on the subject relative to a control with no device operating, based on five replicates.

**Example 1 - Delivery Kinetics**

**a. Delivery kinetics for the commercial 10 mg prallethrin mat**

The rate of release of active from the current article of commerce was determined by running mats in the heater unit for varying periods of time up to 12 hours, then analysing for the active. Three replicates were performed for each time point, and the results are shown in Figure 2 and Table 2.

For the current commercial product, the active decays exponentially, with a decay constant of 0.574 hr$^{-1}$, or a half life of 1.2 hours. This behaviour is maintained over at least three decades of concentration, and is indicative of a simple first order loss process.

**Table 2**. Active decay from 10 mg G0 prallethrin mats.

| Heating time /hr | Mat 1 | Mat 2 | Mat 3 | Average /mg |
|---|---|---|---|---|
| 0 | 12.46 | 10.710 | 8.705 | 10.6 |
| 2 | 5.75 | 2.590 | 3.909 | 4.1 |
| 4 | 1.261 | 1.460 | 0.565 | 1.1 |
| 6 | 0.061 | 0.050 | 0.402 | 0.2 |
| 8 | 0.052 | 0.038 | 0.230 | 0.1 |
| 10 | 0.030 | 0.036 | 0.043 | 0.0 |
| 12 | 0.022 | 0.020 | 0.000 | 0.0 |

**b. Delivery kinetics for the sealed designs G1, G2 and G3.**

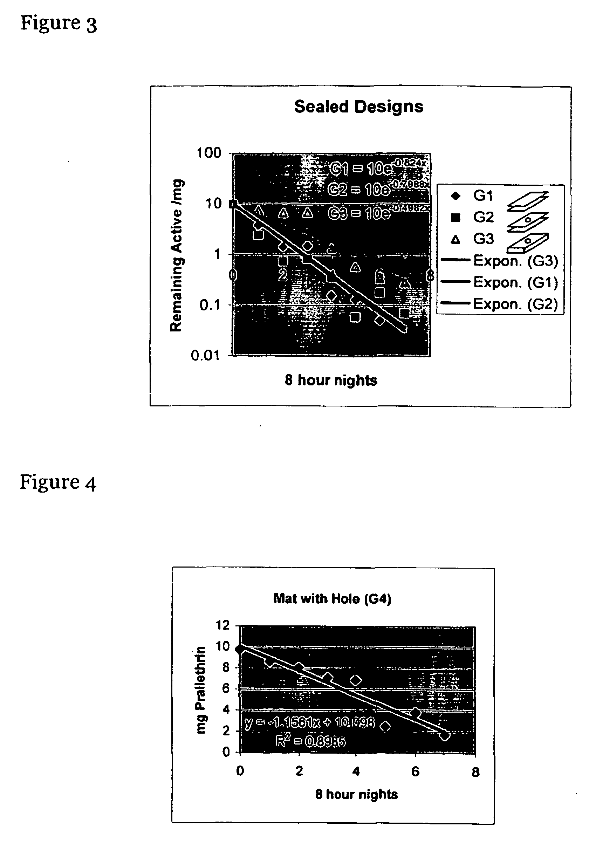

The release rates of active from the sealed designs G1, G2 and G3 were also measured. Examples of the standard 10 mg prallethrin mat were prepared and then wrapped in adhesive metal foil and holes punched so as to create designs G1, G2 and G3. These were also heated on a twelve hour cycle controlled by automatic timers, with eight hours heating and four hours resting, approximating a single nights use. Mats were analysed for active after the requisite number of heating cycles. The results are shown in Figure 3 and Table 3.

These sealed mat designs also show an exponential decay of the active, with half lives for G1, G2 and G3 of 6.7 hrs, 6.9 hrs and 11.1 hrs, respectively (ignoring any contribution from the start/stop effects in the heating cycle). G1 and G2 have a similar performance - the area opened by the punched hole is small to the open area around the edges. But G3, with only the small hole, has a significantly slower release rate.

**Table 3.** Active decay from sealed 10 mg prallethrin mats.

| Heating time /8 hr cycles | G1 | G2 | G3 |
|---|---|---|---|
| 0 | 10 | 10 | 10 |
| 1 | 3.8 | 2.44 | 7.45 |

| Table continued | | | |
|---|---|---|---|
| Heating time /8 hr cycles | G1 | G2 | G3 |
| 2 | 1.42 | 0.73 | 6.67 |
| 3 | 1.49 | 0.83 | 6.78 |
| 4 | 0.16 | 0.36 | 1.35 |
| 5 | 0.13 | 0.06 | 0.59 |
| 6 | 0.05 | 0.18 | 0.34 |
| 7 | 0.07 | 0.07 | 0.28 |

**c. Delivery kinetics for G4.**

The release rate of active from the sealed design G4 was determined over a 7 night period. This design is similar to G3, but the hole is only punched through the top seal instead of being punched through the entire assembly. Surprisingly, the active release from this design was linear, not exponential. The release kinetics are detailed in Figure 4 and Table 4.

**Table 4.** Active decay from sealed 10 mg prallethrin mat.

| Heating time /8 hr cycles | G4 |
|---|---|
| 0 | 9.75 |
| 1 | 8.63 |
| 2 | 8.03 |
| 3 | 7.09 |
| 4 | 6.89 |
| 5 | 2.48 |
| 6 | 3.79 |
| 7 | 1.75 |

**d. Delivery kinetics for G5**

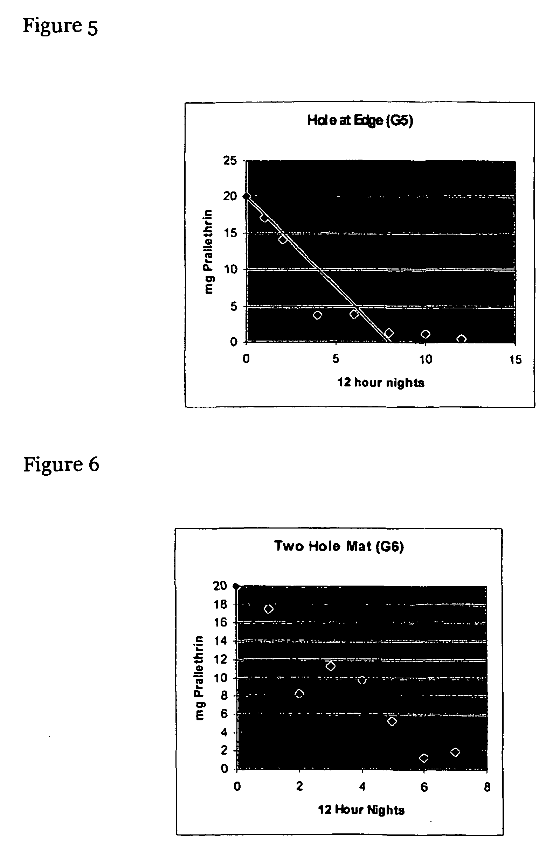

The release rate of active from the sealed design G5 was determined over 12 twelve-hour 'nights'. This design is similar to G4, except that the hole lies not in the centre but towards the edge of the mat. The release kinetics are illustrated in Figure 5. The kinetics are similar to the G4 design, being linear to approximately 7 nights, with a small amount of active remaining for some time thereafter, presumably because it has much further to travel through the mat than in other designs.

**e. Delivery kinetics for G6**

The release rate of active from the sealed design G6 was determined over 7 twelve-hour 'nights'. This design is similar to G5, except that there are two holes, each adjacent to a short edge of the mat. The release kinetics are illustrated in Figure 6. The kinetics are similar to the G4 design, being linear to approximately 7 nights.

**Example 2 - Mass Transport Considerations**

**a. Initial Active Distribution Within a Mat**

There are a number of possible mass transport mechanisms which could determine the rate of active delivery from a mat. The active could evaporate uniformly from the bulk of the mat, or from the top of the mat first, or from the hotter underside first. The active could be released faster from the corners and edges, etc. A knowledge of the mechanisms of mass transport within and out of the mat is necessary if the active release rate is to be controlled. This can be found by analysing the concentration of active in different locations after the mat has been heated for a time.

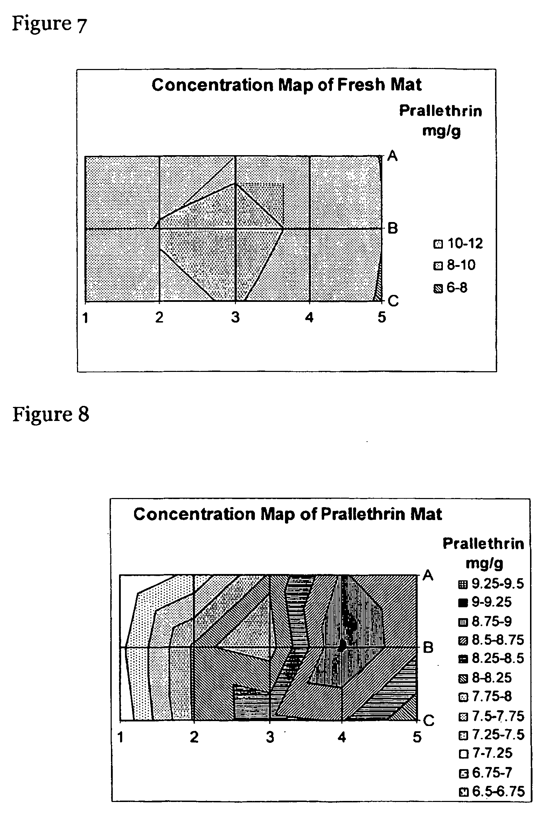

A fresh 10 mg prallethrin mat was cut into 15 segments (3x5 approximately square segments of roughly 5x5 mm each formed by evenly spaced cuts parallel to the edges of the mat). These were analysed to provide an x, y map of active distribution (Figure 7). The initial active distribution is essentially uniform.

**b. Active Movement in an Unsealed Mat**

Two 10 mg prallethrin mats (conventional open configuration, G0) were each heated for two hours in the mat heaters. One was analysed as per the fresh mat, and the active distribution shown in Figure 8. The other was bisected laterally to separate the upper face of the mat from the lower. Analysis gave an indication of the distribution in the z direction.

The contour map of Figure 8 shows an approximately uniform active concentration, with a minor gradient across the face of the mat. This is probably an artefact due to non-uniform heating of the base plate. Other than this the rate of active loss appears roughly uniform across the mat surface.

There is not any preferential loss of active from the edges or corners of the mat, as would be expected if delivery was simply controlled by diffusion from the exterior surfaces of the mat.

However, analysis of the split mat found 96% of the remaining active in the upper half of the mat. The top half of the mat, weighing 0.42 g, held 6.4 mg of active, while the lower half, weighing 0.46 g, held 0.26 mg of active. Now, if the active were simply being vaporised from the bottom up, with no internal movement of the active, the upper portion would be expected to hold 4.8 mg and the lower portion and the lower portion 1.9 mg. The experimental figures indicate a gross movement of active within the mat.

The formulation presumably wicks up and away from the heated plate, probably in response to a heat induced surface tension gradient causing dewetting from the cellulose fibres. The surface tension between two phases depends on the temperature, and with a temperature gradient through the mat, the surface tension of oil against cellulose also changes continuously. Under these conditions the oil can dewet from regions of higher surface energy and wick towards regions of lower surface energy .

**c. Active Movement in a Sealed Mat**

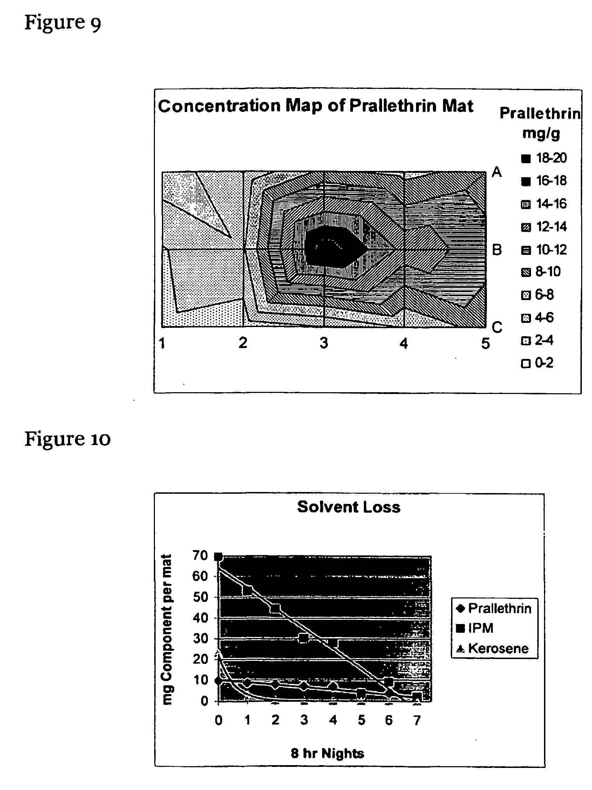

Two sealed 10 mg prallethrin mats (G4 configuration) were each heated for 24 hours (nominally two 12 hour nights) in the mat heaters. One was analysed as per the fresh mat, and the active distribution shown in Figure 9. The other was bisected laterally to separate the upper face of the mat from the lower. Analysis gave an indication of the distribution in the z direction.

Analysis of the split mat found 93% of the remaining active in the upper portion of the mat. The top portion of the mat, weighing 0.60 g, held 6.4 mg of active, while the lower half, weighing 0.48 g, held 0.46 mg of active. This distribution is similar to that observed for the unsealed mat.

The active distribution across the surface is quite non-uniform, with the active concentration peaking in the middle of the mat beneath the hole and falling away to nearly zero at the edges. The active concentration at its peak is approximately double the initial concentration.

The loss of active from the sides could indicate loss through an imperfect seal around the edge. However, if that were the case the concentration in the middle would be no greater than the initial mat concentration. This is not the case.

The overall loss of active could be a result of diffusion of active within the mat towards the hole. However, if that were the case there would be a depletion zone of active below the hole, with the concentration rising out towards the edges. This also is not the case.

The preferred interpretation of this experiment is that the increase in active concentration beneath the hole to a level greater than the initial concentration is a signature of gross liquid movement within the mat, as liquid movement can act to increase the concentration at a given point while diffusion can only decrease the active concentration at any point.

Therefore, it is believed that mass transport in the sealed mat proceeds thus: in response to heating the liquid held within the mat initially moves away from the heat source, and then moves slowly inwards to the area beneath the hole, where release of solvent and active to the atmosphere occurs by evaporation.

**d. Delivery kinetics of the solvents**

The active is dissolved in two solvents - isopropyl myristate ("IPM") and deodorised kerosene. The role of the solvent is critical in mediating the delivery of active, so it is important to understand something of the simultaneous delivery of the solvents.

The loss of the two solvents, along with the active, is shown in Figure 10 (10 mg prallethrin in G4 configuration).

The more volatile kerosene is rapidly lost, and plays no role after about the first day. The isopropyl myristate however decays linearly over the same time frame as the active. The IPM and the active exhaust at nearly the same time. This is probably not accidental - if the mass transport in the mat is dominated by liquid flow, then the active and the IPM should move together.

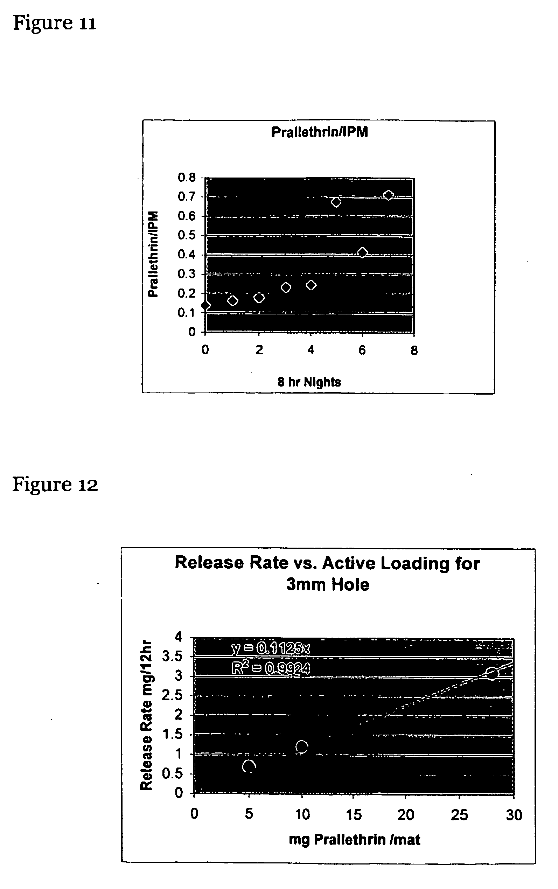

This data may be analysed to see if the active and IPM are delivered rates in proportion to their proportion in the formulation. Figure 11 shows the ratio of prallethrin to IPM over the life of the mat. It shows that there is in fact a significant shift with time, which indicates that the solvent is evaporating at a faster intrinsic rate than the active.

The changing composition of the liquid remaining in the mat is expected to influence the delivery rate. This is because the delivery rate depends on the composition of the vapours produced by the heated mat, which in turn is a function of the composition of the liquid mixture within the mat. Consequently, it is believed that the delivery rate of active may be modulated through the use of solvents of different volatilities, with the use of solvents of lower volatility leading to lower active delivery rates.

### e. Effect of hole size on delivery kinetics

To determine the effect of hole size on the delivery kinetics, three sets of G4 mats were prepared. The physical construction of the mats was as described previously save for the sizes of the holes, which in the first set was 1 mm in diameter, in the second set the usual 3 mm in diameter, and in the third set 6 mm in diameter. The delivery kinetics were substantially linear in the case of the 1 mm and 3 mm holes but has reverted to exponential kinetics in the case of the 6 mm holes. There was little different in the delivery kinetics between the 1 mm and 3 mm holes. Within limits, it appears that the hole size has little effect on the delivery rate. However, once the hole size increases beyond a certain size, the delivery kinetics change from linear to exponential.

### f. Effect of active concentration on delivery kinetics

To determine the effect of active concentration on the delivery kinetics, three sets of G4 mats were prepared. The physical construction of the mats was as described previously. One set was loaded with 5 mg of prallethrin, the second set with 10 mg of prallethrin, and the third set with 30 mg of prallethrin. In all cases the active was delivered in 120mg of formulation. The formulations were based on that given in Table 1, but with the quantities of the non-active ingredients adjusted in so as to be in the same proportions to each other but accommodate the lesser or greater quantity of prallethrin required.

The delivery kinetics were found to be linear in each case. The delivery rate is plotted against active loading in Figure 12. The delivery rate is found to be proportional to the amount of active present. Therefore, increasing the concentration of active in the formulation at constant formulation load does not change the duration of action of the mat, but does change the potency of the insecticidal vapour evolved by the mat.

### Example 3 - Experimental Results - Bioefficacy

The following bioefficacy studies were performed using several different protocols and different insect populations, so care in interpretation is necessary as the bioefficacy measures cannot be directly compared in all cases.

### a. Bioefficacy of the commercial 10 mg prallethrin mat

The bioefficacy of samples of the conventional G0 10 mg prallethrin mat was determined. These mats had been pre-heated for 0.5, 2, 4, 8, 10 and 12 hours prior to testing. The KD50 was measured in a protocol that placed a group of caged mosquitoes (*Culex pipiens*) within a closed glass chamber of volume 0.343 $m^3$, in which the device was operated. Note that mosquito taxonomy has changed, and that *C. pipiens* is an older name for the species now known as *C. quinquefasciatus.*

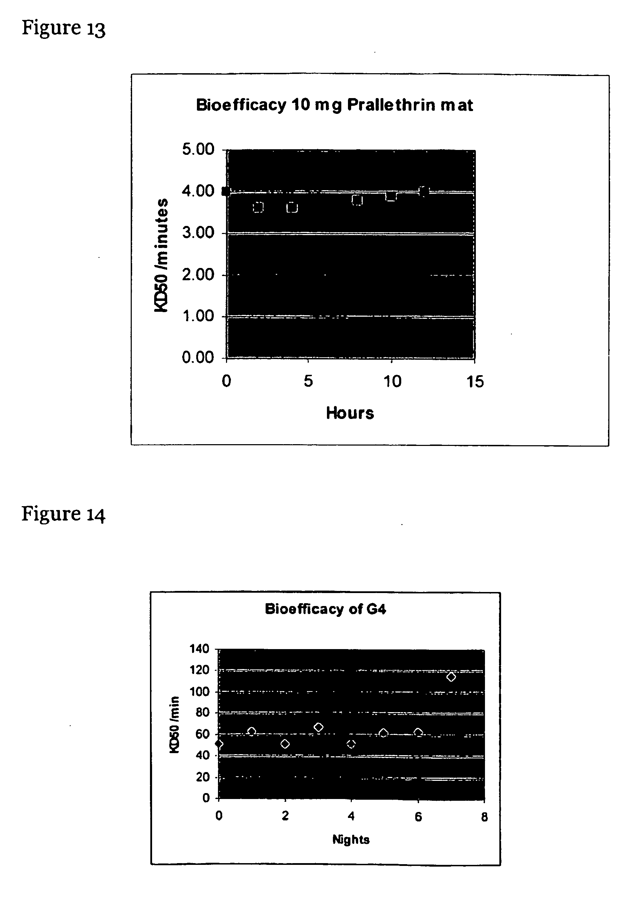

The KD50 of these mats did not change with time (Figure 13), remaining at approximately 4.0 minutes. As the active remaining on the mat has been determined to drop to about 10 $\mu$g at 12 hours (Figure 2), this indicates the mats remain effective with very low levels of active.

This mat was also tested at a single time point (the fresh mat). The KD50 *vs A. Aegypti* was 38 s. Aedes sp. are much more susceptible to insecticides than *Culex* sp. These figures give some idea of the comparison between the two species, although the test protocols are different.

### b. Bioefficacy of Prototype G4 by knockdown time

The bioefficacy of the sealed mat design G4 with 10 mg prallethrin against Aedes *Aegypti* was determined by KD50 measurement in the small chamber protocol. Mats were aged for zero through to seven eight hour nights. The KD50 at each time point is shown in Figure 14, and is a constant 1 minute through to 6 days, with the beginning of a decline in efficacy at the seventh day.

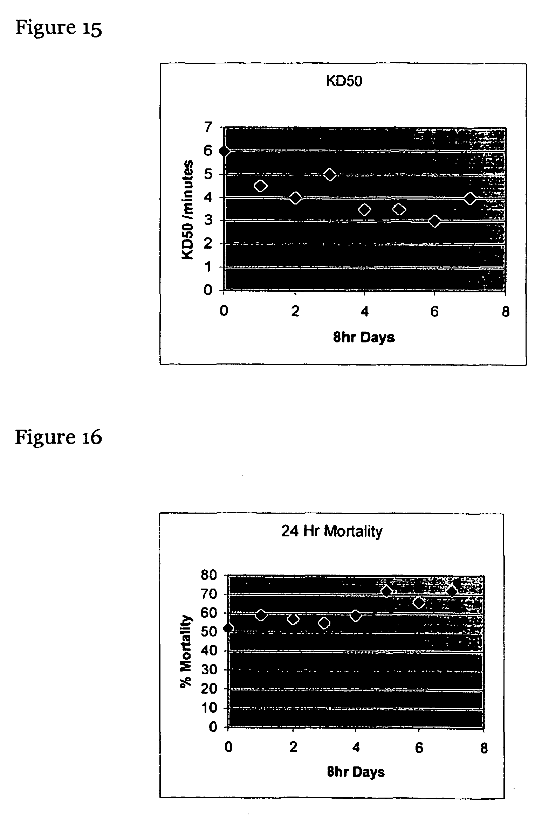

The bioefficacy against *Culex quinquefasciatus* was also established, in the same protocol (Figure 15 and Figure 16). The same mats used for *Aedes* sp. were reused for the *Culex* test, so the time points carry an additional 2 hours, approximately. The KD50 for *Culex* is about 4 minutes, longer than the Aedes knockdown time as is expected for the larger mosquito but identical to the result for the G0 mat, and even shows a slight improvement in efficacy after many hours of use.

**c. Bioefficacy of Prototype G4 by bite inhibition**

Bite inhibition and landing inhibition was determined over 7 days (12 hour heating cycles) for a G4 prototype containing 20 mg of prallethrin. The active was delivered in 120 mg of a formulation based on that of Table 1, but containing 20 mg of active, with the other ingredients reduced proportionately. Also determined was the bite inhibition and landing inhibition of a standard mat (containing 10 mg of active, delivered in 120 mg of the formulation of Table 1). The results are presented in Table 5.

**Table 5.** Bite inhibition of the standard mat and the G4 prototype.

| Treatment | Heating Time | Landing Inhibition | Bite inhibition |
|---|---|---|---|
| Standard Mat | 0 hours | 99.6 | 100% |
| | 3 hours | 99.6 | 100% |
| | 6 hours | 98.0 | 99.9% |
| | 9 hours | 97.5 | 99.9% |
| | 12 hours | 70.2 | 91.9% |
| G4 Prototype | 0 hours | 93.2 | 98.9% |
| | 1x12 hours | 93.1 | 98.9% |
| | 3x12 hours | 95.5 | 99.8% |
| | 5x12 hours | 95.5 | 100% |
| | 7x12 hours | 91.4 | 99% |

Both types of mat give near complete bite inhibition, but while the standard mat is losing its efficacy at 12 hours, the G4 protoype shows continuing efficacy out to at least 7 days.

Discussion

**a. Mass transport within and out of a conventional mat**

The unsealed mat designs (i.e. existing commercial products and related prototypes) all show an exponentially decaying active release rate. Figure 2 exemplifies this, and similar examples can be found through the supplier literature. The exponential decay is quite accurate, extending over at least four decades of concentration.

An exponential decay of this sort indicates a first order loss mechanism, analogous, for instance, to Newton's law of cooling. That is, the rate of active delivery is proportional to the amount of active remaining on the mat,

$$\frac{dM}{dt} = -kM$$

giving

$$M = M_0 \exp(-kt)$$

where M is the mass of active on the mat and k is the decay constant. The half life is

$$t_{1/2} = \ln(2)/k.$$

First order kinetics occurs in many situations. One pertinent situation is evaporation of a volatile solute from an ideal mixture with a non-volatile solvent in a well mixed open vessel. So it is reasonable to conceptualise the vapour delivery from the mat as evaporation out of a bulk solvent, where the solvent may be the solvent in the formulation, or possibly the cellulose fibres of the mat. The basic process consists of a single step:

$$\text{prallethrin}_{\text{MAT}} \rightarrow \text{prallethrin}_{\text{VAPOUR}}.$$

This process will persist through minor and obvious changes to the standard mat design, such as changes in the total amount of active on the mat. A substantially exponential delivery will still result, therefore these simple changes are unlikely to lead to efficient delivery systems capable of the desired long lifetimes. To achieve that end, additional processes must be interposed in the above process. The G4 design introduces an additional process of active movement within the *x-y* plane of the mat to the hole. The active must first move from some location in the mat far from the hole, to a location in the mat beneath the hole, and then escape from the mat as vapour:

$$\text{prallethrin}_{\text{MAT}} \rightarrow \text{prallethrin}_{\text{HOLE}} \rightarrow \text{prallethrin}_{\text{VAPOUR}}$$

This breaks the exponential time dependence of the delivery rate. The overall release kinetics of the more complicated mechanism is substantially linear in time.

### b. Duration of insecticidal action

The various approaches to sealing the mat surfaces were designed in an attempt to interpose further steps into the release process as described above, to determine whether the active release could be linearised, or at least retarded. Figures 2, 3 and 4 show that this is certainly the case, with greater confinement corresponding to slower release.
Most of these results are exponential decays and best characterised by their half lives - these are reported in Table 6. However, G4 follows a fairly linear drop in active level, and a half life is consequently not defined. For this design the 'half-life' quoted is the time for $M_o$ to drop from 10 mg to 5 mg.
Also shown in Table 6 is the open area of the mat - the surface area of cellulose fibre mat exposed to the air. This includes the sides or the internal hole but excludes the area of the face contacting the heater plate.
The half lives for the commercial mats are very short. Even a very large initial dose will not achieve a long duration of action because of the exponential nature of the decay. The half life may be interpreted as the additional lifetime purchased by a doubling of the initial dose. Thus increasing the dose on the commercial mat to 20 mg would only achieve an extra hour of activity.
Likewise, although the half life is longer for designs G1 and G2 the decay is still exponential. The open area around the edge is comparatively large, being about 25% of the exposed area of standard mat, so these designs do not offer much resistance to mass transport out of the mat.
The most interesting design is G4, which does not exhibit an exponential decay, but a linear decay of active, and consequently a constant delivery rate of active. This design therefore has the potential to achieve quite long lifetimes. For instance, doubling the dose of this mat to 20 mg would achieve an extra week, in theory, not just an extra hour as for G0.
The G4 design therefore represents a qualitative departure from existing commercial mat delivery systems, and known prototypes, in that it breaks the exponential decay release profile, allowing a long lasting product to be produced with a modest increase in active loading.
The linear active delivery is a consequence of the geometry. This could be exploited to achieve linear release of fragrances and other volatiles, as well as insecticides.

**Table 6.** Half lives.

| Code | Product | Open Area | Half Life /hr |
|---|---|---|---|
| G0 | Commercial 10 mg prallethrin | 1004 mm$^2$ | 1.2 hrs |
| G1 | 10 mg prallethrin prototype | 228 mm$^2$ | 6.7 hrs |
| G2 | 10 mg prallethrin prototype | 242 mm$^2$ | 6.9 hrs |
| G3 | 10 mg prallethrin prototype | 14 mm$^2$ | 11.1 hrs |

| Table continued | | | |
|---|---|---|---|
| Code | Product | Open Area | Half Life /hr |
| G4 | 10 mg prallethrin prototype | 7 mm$^2$ | 35 hrs[a] |
| [a]Time to 5mg | | | |

**Effective Active Delivery Rates**

**a. Minimum Effective Delivery Rate of Prallethrin and Transfluthrin**

For those experiments where we have both time dependent bioefficacy data and time dependent actives analyses for the mats, we can determine the minimum effective delivery rate for insecticidal action. We currently have two such sets - the experiments with the commercial prallethrin mat (Figures 2 and 10), and the experiments with the G4 design (Figures 4 and 11).

The amount of active remaining on the standard mat (G0) at any time was found to be

$$m(t) = M_o \exp(-0.574\, t)$$

where t is in hours and $M_o$ in this case is 10 mg of prallethrin. The delivery rate at any time is thus

$$\frac{\partial m}{\partial t} = 5.7\exp(-0.574t) \quad \mathrm{mg/hr.}$$

This mat showed a constant (and effective) bioefficacy out to at least 12 hours. At 12 hours, the delivery rate is

$$\frac{\partial m}{\partial t} = 0.006$$

$$\mathrm{mg/hr}$$

This is the lowest verified effective delivery rate for prallethrin. Any device that achieves this delivery rate should give a KD50 versus *Culex pipiens* of about 4 minutes in the knockdown protocol described above.

**b. Delivery rate from the G4 mat design**

The delivery rate of prallethrin can also be calculated for the G4 design. From Figure 4, the delivery rate is a constant

$$\frac{\partial m}{\partial t} = 0.14$$

$$\mathrm{mg/hr}$$

up until exhaustion of active.

To put this figure in perspective, the release rate of the G4 mat is the same as that of a standard mat that has been run for 6.5 hours, ie. a G4 mat is about as efficacious as a half-used standard mat. However, this release rate can be maintained over many days.

**Summary and Conclusions**

This study provides new insights into how insecticidal mats work, their current limitations, and ways to design around those limitations. To briefly summarise:

Current insecticidal mats are very inefficient, wasteful delivery systems. The delivery rate of active decays exponentially with time, with a short half life. Therefore, to achieve one whole night's protection, an enormous amount of active must be used. Most of this is released very early in the operation of the mat, but this waste is necessary to achieve activity out to the required time of 12 hours.

Based on the known least effective release rate, an efficient delivery system could use 1% of the amount of active presently used, and still provide an effective product.

For the same reason, current mat designs cannot deliver a long lasting mat. The amount of active required increases exponentially with the desired lifetime. The amount of active that would be required for a 7 day mat would make such a product prohibitively expensive.

An efficient delivery system is therefore needed. In this case, 'efficient' means that the device can emit active at a rate just above the least effective rate for long periods of time. In other words, a constant delivery rate must be achieved, i.e. linear delivery kinetics are required.

One way to retard active delivery would be to partially seal the mat within an impermeable barrier. Mats with four different barrier geometries were prepared (G1-G4). All showed a retarded release rate, but G1-G3 still showed an exponential delivery rate. For the reasons given above, these will not be efficient design for long life.

However, the design G4 with the most restrictive barrier showed a linear release rate. The barrier design has changed the fundamental release mechanism, and this design is capable of efficiently delivering active over a long lifetime. The first prototype was shown to deliver active at the same rate for about 8 days, before exhaustion.

Without wishing to be bound by theory, the likely explanation for the change in release kinetics is that the geometry of the barrier design rather than its total area is the key. The mathematics of gradient driven mass transport in a radial geometry is different to a rectilinear geometry. A simple scaling geometrical scaling argument elicits the linear form of the release.

The G4 design was tested for efficacy against two species of mosquitoes - *Aedes aegypti* and *Culex quinquefasciatus.* In each case, a constant and effective bioefficacy over about 7 days was observed. This was all the more remarkable because the amount of active used was the same as used in the current single night mat. This design is therefore suitable for development as a commercial product.

Returning to the bioefficacy experiments, in some cases where we have paired kinetic and bioefficacy data we can estimate the minimum delivery rates required for efficacy. This seems to be much lower than commonly held. For instance, coils deliver an equivalent of order about 1 mg/hr of prallethrin. S.C. Johnson claim the effective delivery rate for transfluthrin (of similar activity to prallethrin) is 0.2 mg/hr.

However, by analysing the delivery rate of an effective standard mat at its end of life, we find a delivery rate of 0.006 mg/hr is still effective. This discrepancy in rates can be exploited for significant margin improvement, by reducing active use accordingly. Knowing the minimum effective rate can also be used to design a long life product if the release kinetics are understood.

The kinetic analysis also shows that the G4 mat is approximately equivalent to a standard mat after 6 hours heating, i.e. at the mid point of its lifetime.

From all the above, it follows that there is significant scope for optimizing existing and quite old mat designs, achieving greater economy of active use and longer durations of effect at the same time.

various modifications and variations of the described methods and devices of the invention will be apparent to those skilled in the art without departing from the scope of the invention. Although the invention has been described in connection with specific preferred embodiments, it should be understood that the invention as claimed, should not be unduly limited to such specific embodiments. Indeed, various modifications of the described modes for carrying out the invention which are apparent to those skilled in the relevant are are intended to be within the scope of the following claims.

**Claims**

1. A substance delivery device comprising (i) a substrate layer (2) impregnated with one or more active substances, (ii) an outer layer (1) which encloses the substrate layer and is substantially impermeable to the one or more active substances, and (iii) one or more apertures (3) in the outer layer which permit release of the one or more active substances from the substrate layer into the atmosphere, the apertures being present only in a portion of the outer layer which in use is exposed to the atmosphere, **characterised in that** the substrate layer is in direct fluid communication with the atmosphere, and that the total surface area of the apertures is less than 2% of the total surface area of the outer layer such that the one or more active substances are released into the atmosphere at a substantially linear rate.

2. A device according to claim 1 wherein the total surface area of the apertures is less than 1% of the total surface area of the outer layer.

3. A device according to claim 1 or 2 wherein the substrate layer further comprises one or more solvents.

4. A device according to claim 3 wherein at least one of the solvents has a molar enthalpy of vaporisation greater than $3.77 \times 10^3$ J/mol (9 kcal/mol).

5. A device according to any one of claims 1 to 3 wherein the one or more active substances in the substrate layer is/are vaporisable upon heating of the substrate layer.

6. A device according to any one of the preceding claims wherein at least one of the apertures is present substantially in the centre of said portion which in use is exposed to the atmosphere.

7. A device according to claim 6 wherein there is only one aperture in the outer layer.

8. A device according to any one of the preceding claims where the substrate layer comprises one or more holes which are in at least partial alignment with the one or more apertures.

9. A device according to claim 8 wherein the hole(s) extend partially through the substrate layer.

10. A device according to claim 8 wherein the hole(s) extend completely through the substrate layer.

11. A device according to any one of the preceding claims which is substantially flat.

12. A device according to claim 11 wherein the substrate layer is a fibrous mat.

13. A device according to any one of the preceding claims wherein at least one of the one or more active substances is an insecticidal substance.

14. A device according to claim 13 wherein the insecticidal substance is a pyrethroid.

15. A device according to claim 13 or 14 wherein the substrate layer comprises an indicator layer comprising a material the light transmitting properties of which vary depending on the amount of solvent present in the substrate.

16. A device according to any one of the preceding claims further comprising removable sealing means for sealing said apertures prior to use.

17. A method for producing a device according to any one of claims 1 to 16 which method comprises sealing a substrate layer comprising one or more active substances in an outer layer which is impermeable to the one or more substances; and introducing one or more apertures in the outer layer

18. A method for producing a device according to any one of claims 1 to 16 which comprises sealing a substrate layer comprising one or more active substances in an outer layer which is impermeable to the one or more substances and which comprises one or more apertures.

19. A method according to claim 17 or 18 which further comprises sealing said one or more apertures with a removable sealing means.

20. A method for releasing one or more active substances into the atmosphere which method comprises heating the device of any one of claims 1 o 16 so as to cause release of the one or more active substances into the atmosphere via the apertures in the outer layer.

21. A method for inhibiting insect biting which method comprises heating the device of any one of claims 1 to 16, wherein at least one of the one or more active substances is an insecticidal substance, so as to cause release of the one or more insecticidal substances into the atmosphere via the apertures in the outer layer.

**Patentansprüche**

1. Stoffabgabevorrichtung, umfassend (i) eine Substratschicht (2), die mit einem oder mehreren Wirkstoffen imprägniert ist, (ii) eine Außenschicht (1), die die Substratschicht umschließt und für den einen oder die mehreren Wirkstoffe undurchlässig ist, und (iii) eine oder mehrere Öffnungen (3) in der Außenschicht, die die Abgabe des einen oder der mehreren Wirkstoffe von der Substratschicht in die Atmosphäre gewähren, wobei die Öffnungen beim Gebrauch nur in einem bei Verwendung der Atmosphäre ausgesetzten Teil der Außenschicht vorliegen, **dadurch gekennzeichnet, dass** die Substratschicht in direkter Fließkommunikation mit der Atmosphäre steht, und dass der gesamte Oberflächenbereich der Öffnungen weniger als 2% des gesamten Oberflächenbereichs der Außenschicht beträgt, sodass der eine oder die mehreren Wirkstoffe in die Atmosphäre mit im Wesentlichen linearer Geschwindigkeit in die Atmosphäre abgegeben werden.

2. Vorrichtung nach Anspruch 1, wobei der gesamte Oberflächenbereich der Öffnungen weniger als 1% des gesamten Oberflächenbereichs der Außenschicht beträgt.

3. Vorrichtung nach Anspruch 1 oder 2, wobei die Substratschicht des Weiteren ein oder mehrere Lösungsmittel umfasst.

4. Vorrichtung nach Anspruch 3, wobei mindestens eines der Lösungsmittel eine molare Verdampfungsenthalpie von größer als $3{,}77 \times 10^3$ J/mol (9 kcal/mol) aufweist.

5. Vorrichtung nach einem der Ansprüche 1 bis 3, wobei der eine oder die mehreren Stoffe in der Substratschicht beim Erwärmen der Substratschicht verdampfungsfähig ist/sind.

6. Vorrichtung nach einem der vorangehenden Ansprüche, wobei mindestens eine der Öffnungen im Wesentlichen in der Mitte des bei Verwendung der Atmosphäre ausgesetzten Teils vorliegt.

7. Vorrichtung nach Anspruch 6, wobei nur eine Öffnung in der Außenschicht vorliegt.

8. Vorrichtung nach einem der vorangehenden Ansprüche, wobei die Substratschicht ein oder mehrere Löcher umfasst, die zumindest teilweise mit den einen oder mehreren Öffnungen übereinstimmen.

9. Vorrichtung nach Anspruch 8, wobei sich das Loch bzw. die Löcher teilweise durch die Substratschicht erstreckt bzw. erstrecken.

10. Vorrichtung nach Anspruch 8, wobei sich das Loch bzw. die Löcher vollständig durch die Substratschicht erstreckt bzw. erstrecken.

11. Vorrichtung nach einem der vorangehenden Ansprüche, die im Wesentlichen flach ist.

12. Vorrichtung nach Anspruch 11, wobei die Substratschicht eine Fasermatte ist.

13. Vorrichtung nach einem der vorangehenden Ansprüche, wobei mindestens einer der einen oder mehreren Wirkstoffe ein insektizider Stoff ist.

14. Vorrichtung nach Anspruch 13, wobei der insektizide Stoff ein Pyrethroid ist.

15. Vorrichtung nach Anspruch 13 oder 14, wobei die Substratschicht eine Indikatorschicht umfasst, die ein Material umfasst, dessen lichtdurchlässige Eigenschaften je nach im Substrat vorliegender Lösungsmittelmenge variieren.

16. Vorrichtung nach einem der vorangehenden Ansprüche, des Weiteren umfassend entfernbare Versiegelungsmittel zum Versiegeln der Öffnungen vor der Verwendung.

17. Verfahren zur Herstellung einer Vorrichtung nach einem der Ansprüche 1 bis 16, wobei das Verfahren das Versiegeln einer Substratschicht, umfassend einen oder mehrere Wirkstoffe in einer für den einen oder die mehreren Wirkstoffe undurchlässigen Außenschicht; und Einbringen einer oder mehrer Öffnungen in die Außenschicht umfasst.

18. Verfahren zur Herstellung einer Vorrichtung nach einem der Ansprüche 1 bis 16, das das Versiegeln einer Substratschicht, umfassend einen oder mehrere Wirkstoffe in einer für den einen oder die mehreren Wirkstoffe undurchlässigen und eine oder mehrere Öffnungen umfassenden Außenschicht, umfasst.

19. Verfahren nach Anspruch 17 oder 18, das des Weiteren das Versiegeln der einen oder mehreren Öffnungen mit einem entfernbaren Versiegelungsmittel umfasst.

20. Verfahren zur Abgabe von einem oder mehreren Wirkstoffen in die Atmosphäre, wobei das Verfahren das derartige Erwärmen der Vorrichtung nach einem der Ansprüche 1 bis 16 umfasst, dass die Abgabe des einen oder der mehreren Wirkstoffe in die Atmosphäre über die Öffnungen in der Außenschicht bewirkt wird.

21. Verfahren zum Hemmen von Insektenbissen, wobei das Verfahren das derartige Erwärmen der Vorrichtung nach einem der Ansprüche 1 bis 16 umfasst, wobei mindestens einer des einen oder der mehreren Wirkstoffe ein insektizider Stoff ist, dass die Abgabe des einen oder der mehreren insektiziden Stoffe in die Atmosphäre über die Öffnungen in der Außenschicht bewirkt wird.

## Revendications

1. Dispositif de distribution de substance comprenant (i) une couche servant de substrat (2) imprégnée d'une ou plusieurs substances actives, (ii) une couche externe (1) renfermant la couche servant de substrat et essentiellement imperméable à ladite au moins une substance active, et (iii) une ou plusieurs ouvertures (3) dans la couche externe qui permettent le passage de ladite au moins une substance active depuis la couche servant de substrat jusque dans l'atmosphère, les ouvertures n'étant présentes que dans une partie de la couche externe qui, lors de l'utilisation, est exposée à l'atmosphère, le dispositif étant **caractérisé en ce que** la couche servant de substrat est en communication de fluide directe avec l'atmosphère et **en ce que** l'aire de surface totale des ouvertures représente moins de 2 % de l'aire de surface totale de la couche externe, si bien que ladite au moins une substance active est libérée dans l'atmosphère de façon essentiellement linéaire.

2. Dispositif selon la revendication 1, dans lequel l'aire de surface totale des ouvertures représente moins de 1 % de l'aire de surface totale de la couche externe.

3. Dispositif selon la revendication 1 ou 2, dans lequel la couche servant de substrat comprend, en outre, un ou plusieurs solvants.

4. Dispositif selon la revendication 3, dans lequel au moins l'un des solvants présente une enthalpie molaire de vaporisation supérieure à 3,77 x $10^3$ J/mol (9 kcal/mol).

5. Dispositif selon l'une quelconque des revendications 1 à 3, dans lequel ladite au moins une substance active, présente dans la couche servant de substrat, est vaporisable suite au chauffage de la couche servant de substrat.

6. Dispositif selon l'une quelconque des revendications précédentes, dans lequel au moins l'une des ouvertures est présente essentiellement au centre de ladite partie qui, lors de l'utilisation, est exposée à l'atmosphère.

7. Dispositif selon la revendication 6, dans lequel une seule ouverture est présente dans la couche externe.

8. Dispositif selon l'une quelconque des revendications précédentes, dans lequel la couche servant de substrat comprend un ou plusieurs orifices qui sont au moins partiellement alignés avec ladite au moins une ouverture.

9. Dispositif selon la revendication 8, dans lequel l'/les orifice(s) se prolonge(nt) partiellement à travers la couche servant de substrat.

10. Dispositif selon la revendication 8, dans lequel l'/les orifice(s) se prolonge(nt) entièrement à travers la couche servant de substrat.

11. Dispositif selon l'une quelconque des revendications précédentes, qui est essentiellement aplati.

12. Dispositif selon la revendication 11, dans lequel la couche servant de substrat est une plaquette fibreuse.

**13.** Dispositif selon l'une quelconque des revendications précédentes, dans lequel ladite au moins une substance active est une substance insecticide.

**14.** Dispositif selon la revendication 13, dans lequel la substance insecticide est un pyréthroïde:

**15.** Dispositif selon les revendications 13 ou 14, dans lequel la couche servant de substrat comporte une couche indicatrice comprenant un matériau dont les propriétés de transmission de la lumière varient en fonction de la quantité de solvant présente dans le substrat.

**16.** Dispositif selon l'une quelconque des revendications précédentes, comprenant, en outre, un moyen d'obturation amovible destiné à obturer lesdites ouvertures avant utilisation.

**17.** Procédé de fabrication d'un dispositif selon l'une quelconque des revendications 1 à 16, ledit procédé comprenant le scellage d'une couche servant de substrat comportant une ou plusieurs substances actives dans une couche externe imperméable à ladite au moins une substance ; et la création d'une ou de plusieurs ouvertures dans la couche externe.

**18.** Procédé de fabrication d'un dispositif selon l'une quelconque des revendications 1 à 16, comprenant le scellage d'une couche servant de substrat comportant une ou plusieurs substances actives dans une couche externe imperméable à ladite au moins une substance et comprenant une ou plusieurs ouvertures.

**19.** Procédé selon les revendications 17 ou 18, comprenant, en outre, l'obturation de ladite au moins une ouverture à l'aide d'un moyen d'obturation amovible.

**20.** Procédé de libération d'une ou plusieurs substances actives dans l'atmosphère, ledit procédé comprenant le chauffage du dispositif selon l'une quelconque des revendications 1 à 16, de façon à provoquer la libération de ladite au moins une substance active dans l'atmosphère par l'intermédiaire des ouvertures pratiquées dans la couche externe.

**21.** Procédé destiné à éviter les piqûres d'insectes, ledit procédé comprenant le chauffage du dispositif selon l'une quelconque des revendications 1 à 16, dans lequel au moins une de ladite au moins une substance active est une substance insecticide, de façon à provoquer la libération de ladite au moins une substance insecticide dans l'atmosphère par l'intermédiaire des ouvertures pratiquées dans la couche externe.

## Figure 1

| Code | Illustration | Description |
|------|-------------|-------------|
| G0 | | Bare mat, current commercial product |
| G1 | | Mat sealed on top and bottom surfaces |
| G2 | | Mat sealed on top and bottom surfaces, with a hole punched completely through all layers |
| G3 | | Mat sealed on all surfaces, with a hole punched completely through all layers |
| G4 | | Mat sealed on all surfaces, with a hole punched through top layer only |

## Figure 2

Release from Standard (G0) Mat

$mg\ Prallethrin = 10.6e^{-0.574hr}$

$R^2 = 0.9842$

## Figure 3

Sealed Designs

## Figure 4

Mat with Hole (G4)

Figure 5

Figure 6

## Figure 7

**Concentration Map of Fresh Mat**

Prallethrin
mg/g

A

B

C

1    2    3    4    5

☐ 10-12
☐ 8-10
☒ 6-8

## Figure 8

**Concentration Map of Prallethrin Mat**

Prallethrin
mg/g

A

B

C

1    2    3    4    5

☐ 9.25-9.5
■ 9-9.25
☐ 8.75-9
☒ 8.5-8.75
■ 8.25-8.5
☒ 8-8.25
☐ 7.75-8
☒ 7.5-7.75
☐ 7.25-7.5
☐ 7-7.25
☒ 6.75-7
☒ 6.5-6.75

Figure 9

**Concentration Map of Prallethrin Mat**

Prallethrin mg/g

- 18-20
- 16-18
- 14-16
- 12-14
- 10-12
- 8-10
- 6-8
- 4-6
- 2-4
- 0-2

Figure 10

**Solvent Loss**

- Prallethrin
- IPM
- Kerosene

mg Component per mat

8 hr Nights

## Figure 11

## Figure 12

## Figure 13

**Bioefficacy 10 mg Prallethrin mat**

## Figure 14

**Bioefficacy of G4**

## Figure 15

KD50

## Figure 16

24 Hr Mortality

Figure 17

(3)

(2)

(1)

Figure 18

(3)

(2)

(1)

Figure 19

(3)

(2)

(1)